# EUROPÄISCHE PATENTSCHRIFT

(11) **EP 0 396 871 B1**
(45) Veröffentlichungstag und Bekanntmachung des Hinweises auf die Patenterteilung: **01.06.1994**
(21) Anmeldenummer: 90104654.0
(22) Anmeldetag: 12.03.1990
(51) Int. Cl.: C07C 229/12, C11D 1/88, A61K 7/06, A61K 7/48

(54) **Neue N-Alkylglykaminoverbindungen sowie ein Verfahren zur Herstellung und ihre Verwendung**
N-Alkylglycamino compounds, a method for their preparation and their use
Composés du type N-alkylglycamino, un procédé pour leur préparation et leur utilisation

(30) Priorität: 09.05.1989 DE 3915121
(43) Veröffentlichungstag der Anmeldung: 14.11.1990
(73) Patentinhaber: HÜLS AKTIENGESELLSCHAFT, 45764 Marl (DE)
(72) Erfinder: Kelkenberg, Heike, Dr., D-4390 Gladbeck (DE)

(56) Entgegenhaltungen:
- DE-A- 3 711 776
- DE-C- 950 289
- FR-A- 1 573 085
- CHEMICAL ABSTRACTS, Band 62, Nr. 10, 10. Mai 1965, Spalte 11675, Zusammenfassung Nr. 11675 a-b, Columbus, Ohio, US; E. ULSPERGER et al.:"Surface-active polyhydroxy compounds. XX. Compounds resulting from the addition reaction of C8-12 normal fatty amines and glycidol"

## Beschreibung

Die Erfindung betrifft neue N-Alkylglykaminoverbindungen, sowie ein Verfahren zur Herstellung und ihre Verwendung.

Nachwachsende Rohstoffe sind wichtige Rohstoffe für den Waschmittel- und Körperpflegebereich. Neben den pflanzlichen Ölen und tierischen Fetten gewinnen Stärke und Monosaccharide zunehmend an Bedeutung. Die ersten Polyglycoside und Saccharidester werden bereits als umweltfreundliche Tenside auf dem Markt angeboten. Glykamine auf Monosaccharid-Basis stellen ebenfalls gute Ausgangsstoffe für waschaktive und körperpflegende Anwendungen dar. So werden z. B. tertiäre und quartäre Fettalkylglykammonium-Salze als kationaktive Reinigungsmittel erwähnt (US-PSS 2 060 850, 2 060 851). Entsprechende oxethylierte Verbindungen werden als schwachschäumende Spül- und Reinigungsmittel sowie als Haut- und Haarpflegemittel vorgeschlagen (FR-PS 1 573 085, DE-OS 22 00 040, JP 59 212 419, JP 59 212 421). Ebenfalls bekannt sind nichtionische Detergentien auf Basis Fettsäure-N-alkylglykamiden, die besonders hautschonend sind und gleichzeitig verdickend wirken auf Shampoo- und Duschbad-Formulierungen (US-PS 2 703 798, DE-OS 37 11 776).

Von Ulsperger und Dehns [J. Prakt. Chem. 27, 195 (1965)] werden grenzflächenaktive Additionsprodukte aus n-Fettaminen der Kettenlänge C₈-C₁₂ und Glycid beschrieben. Die Amine lassen sich durch Umsetzung mit Chloressigsäure als kristalline Na-N-Alkyl-N-[β,γ-dihydroxypropyl)-glycinate HOCH₂CH(OH)CH₂NC₂H₂ₙ₊₁CH₂COONa charakterisieren.

Amphotere Tenside stellen eine Stoffklasse dar, die je nach Milieueinstellung (sauer, neutral oder alkalisch) sich wie anionische oder kationische Tenside verhalten, d. h. sie haben elektronegative und elektropositive Bausteine im gleichen Molekül vereinigt. Sie gewinnen zunehmend im Körperpflegebereich Bedeutung, da sie eine besonders gute Hautverträglichkeit und eine geringe Toxizität aufweisen und dabei in schwach saurem Medium als mildes Tensid gute Wirkung zeigen.

Im allgemeinen handelt es sich hierbei um N-substituierte Aminosäuren folgenden Typs (Formel I): 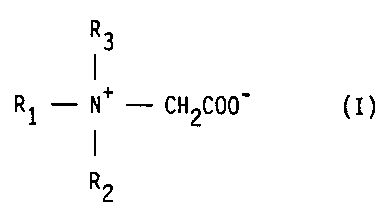
in dem R₁ für eine längere Fettalkylkette und R₂ z. B. für einen kürzeren Alkyl-Rest stehen. R₃ kann sowohl ein Proton oder einen Alkyl-Rest darstellen. In ihrem Verhalten wird deutlich zwischen den Aminosäuren mit tetrasubstituiertem Stickstoff und denen mit trisubstituiertem Stickstoff unterschieden:
Die tetrasubstituierten Aminosäuren werden auch Betaine (Formel II) genannt. Sie besitzten unabhängig vom pH des Milieus eine fixierte positive Ladung. Im Neutral- und Alkalibereich bleibt der isoelektrische, zwitterionische Charakter erhalten; im sauren Bereich verhält sich das Betain wie ein kationisches Tensid. 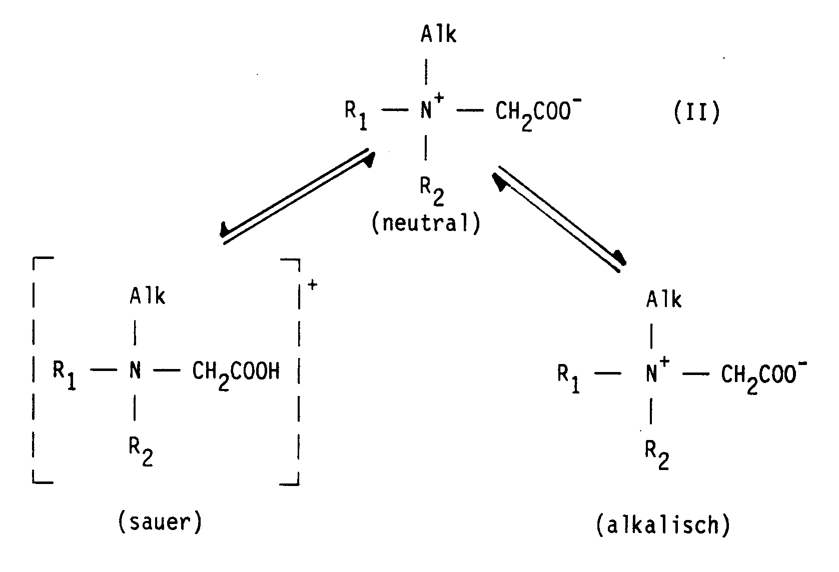
Die trisubstituierten Aminosäuren (Formel III) dagegen stellen echte Ampholyte dar. 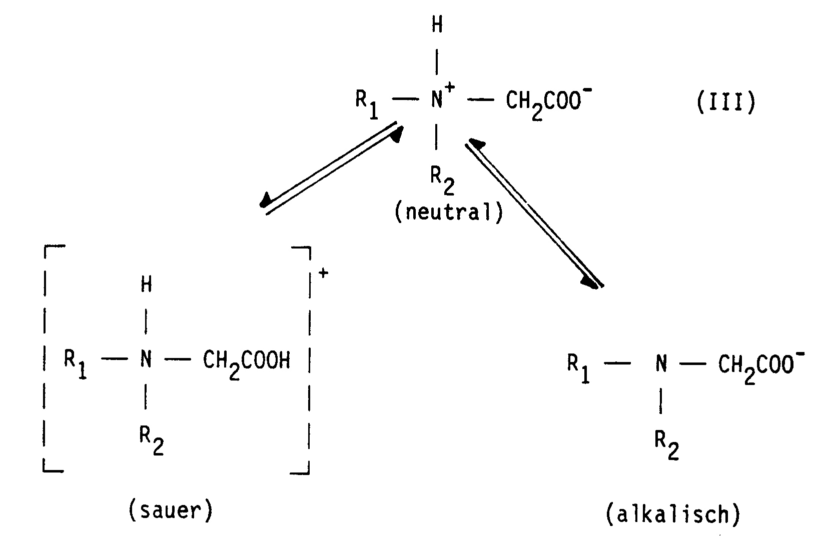
Im Neutralbereich bilden sie innere Salze; im sauren Medium gehen sie in Kationen, im alkalischen Bereich in Anionen über.

Ein Nachteil für die bekannten N-trisubstituierten Amphotenside ist, daß sie im gewünschten pH-Bereich, nämlich schwach sauer bis neutral, bei der Anwendung im Körperpflegebereich, wie Haar- und Duschshampoos, ein Minimum in der Löslichkeit, des Schaumvermögens und des Benetzungsvermögens aufweisen [Tenside Detergents 23 (1986) 309].

Der Erfindung liegt daher die Aufgabe zu Grunde, verbesserte N-trisubstituierte Amphotenside auf Basis von Monosacchariden als nachwachsende Rohstoffe aufzufinden, die zudem die vorgenannten Nachteile nicht aufweisen.

Diese Aufgabe wurde erfindungsgemäß durch die Glykaminoessigsäure/acetate der Formel IV gelöst. 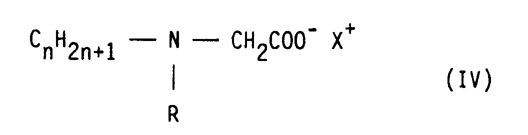
Der Gegenstand der Erfindung sind daher N-Alkylglykaminoverbindungen der allgemeinen Formel IV 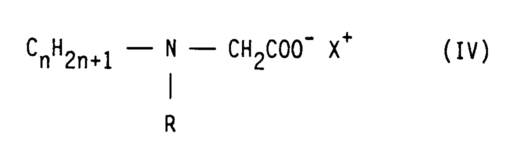
in der
n 4 - 24, und 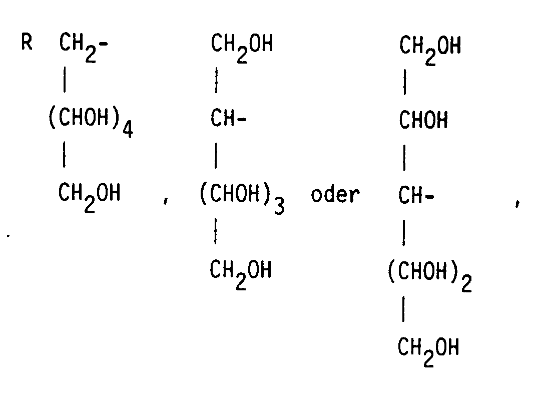
und
X Na, K oder H
bedeuten.

N-Alkyl-N-(desoxyhexityl)-essigsäure-Salze entsprechen der allgemeinen Formel IV 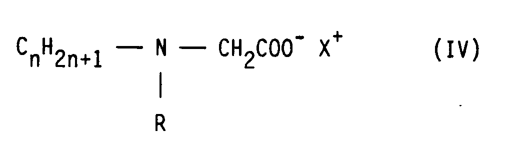
in der n gleich 4 - 24, bevorzugt 8 - 14, sein kann und R vorzugsweise für einen Desoxyhexityl-Rest steht, wie z. B. 1-Desoxy-1-glucityl-, 2-Desoxy-2-glucityl-, 3-Desoxy-3-glucityl-, 1-Desoxy-1-mannityl-, 2-Desoxy-2-mannityl-, 3-Desoxy-3-mannityl-, 1-Desoxy-1-dulcityl-, 2-Desoxy-2-dulcityl-, 3-Desoxy-3-dulcityl-, 1-Desoxy-1-allityl-, 2-Desoxy-2-allityl-, 3-Desoxy-3-allityl-, 1-Desoxy-1-altrityl-, 2-Desoxy-2-altrityl-, 3-Desoxy-3-altrityl-, 1-Desoxy-1-talityl-, 2-Desoxy-2-talityl- oder 3-Desoxy-3-talityl-; vorzugsweise steht R für einen 1-Desoxy-1-glucityl-Rest, sowie X bevorzugt für Na.

Es wurde nun überraschend gefunden, daß diese erfindungsgemäßen trisubstituierten, amphoteren Verbindungen der Formel IV hervorragende tensidische Eigenschaften aufweisen. Sie sind im Vergleich zu trisubtituierten, amphoteren Tensiden der Formel III gut wasserlöslich. Außerdem zeigen die erfindungsgemäßen trisubstituierten Amphotenside eine gute Hautverträglichkeit. Dabei zeigt insbesondere das C₁₂-Alkylglucaminoacetat herausragende Eigenschaften: im gesamten pH-Bereich von 2 bis 12 tritt keine Opaleszenz auf, sondern es ist klar löslich.

Die Herstellung von chemischen Verbindungen der Formel IV erfolgt durch Carboxymethylierung von N-Alkylglykaminen, wie z. B. N-Alkyl-1-Amino-1-desoxyhexitylen, nach allgemein bekanntem Verfahren je nach Löslichkeit des Amins in Wasser oder Butanol als Lösemittel. Bei Einhaltung des pH-Bereiches von 8 - 9 durch kontinuierliche Zugabe von Alkali und Einhaltung einer Reaktionstemperatur von kleiner 80 °C wird so eine selektive N-Carboxymethylierung erreicht, ohne daß die OH-Gruppen des Hexityl-Restes reagieren.

Die N-Alkylglykamine selbst werden nach allgemein bekanntem Verfahren der reduktiven Aminierung von Monosacchariden (US-PSS 1 994 467, 2 016 962), insbesondere Hexosen mit Alkylaminen wie z. B. Butylamin, Octylamin, Laurylamin, Myristylamin, Stearylamin oder Kokosfettamin hergestellt, wobei als Beispiele der Monosaccharide zu nennen sind: Glucose, Galaktose, Mannose, Fructose, Allose, Altrose, Talose sowie Glucose-Sirup.

Die Erfindung soll durch folgende Beispiele erläutert werden.

### Herstellungsbeispiele

### 1. Octylglucaminoessigsäure-Na-Salz

19,4 g (0,205 mol) Chloressigsäure werden in 106,2 g (5,9 mol) Wasser gelöst und 58,6 g (0,2 mol) Octylglucamin portionsweise unter Rühren hinzugegeben. Gleichzeitig wird die Temperatur auf 60 °C erhöht. Nach vollständiger Auflösung des Octylglucamins werden 102,4 g (88 ml, 0,409 mol) einer 16%igen Natronlauge bei einem pH-Wert zwischen 8 und 9 zugetropft. Gegen Ende der Reaktion wird die Temperatur auf 65 °C erhöht. Nach Beendigung der Natronlaugezugabe läßt man 1 h bei 70 °C nachreagieren. In der Kälte fällt das nichtumgesetzte Octylglucamin (6,5 g, Fp: 121 - 123 °C, Basenzahl: 183,6 mg KOH/g) aus und wird abfiltriert. Das Filtrat wird anschließend zum Trocknen eingeengt.

| | |
|---|---|
| Ausbeute: | 78,6 g (83,2 %) |
| Schmelzbereich: | 80 - 154 °C |
| Stickstoffgehalt: | 3,01 % (3,75 %) |
| Carboxyl-Zahl: | 121 mg KOH/g |
| Reinheit: | 79 % Octylglucaminoessigsäure-Na (¹³C-NMR) |
| | 6 % Na-Glykolat |
| | 15 % NaCl |

### 2. Decylglucaminoessigsäure-Na-Salz

19,4 g (0.205 mol) Chloressigsaure werden in 192,6 g (2,6 mol) n-Butanol gelöst und 64.2 g (0,2 mol) Decylglucamin portionsweise unter Ruhren hinzugegeben. Dabei wird die Temperatur auf 60 °C erhöht. Nach vollständiger Auflösung des Decylglucamins werden 102,4 g (88 ml, 0,409 mol) einer 15%igen Natronlauge bei einem pH-Wert zwischen 8 und 9 zugetropft. Gegen Ende der Reaktion wird die Temperatur auf 65 °C erhöht. Nach Beendigung der Natronlaugezugabe läßt man 1 h bei 70 °C nachreagieren. Anschließend wird das Wasser unter kontinuierlicher n-Butanolzugabe azeotrop abdestilliert. Dabei tritt eine Fällung von NaCl auf. Diese wird heiß abfiltriert. Aus diesem Filtrat tritt in der Kälte ein weißer Niederschlag auf.

Der abgetrennte Niederschlag wird im Verhältnis 1 : 3 heiß in Wasser gelöst und anschließend gekühlt. Dabei fällt das nicht umgesetzte Decylglucamin (2,5 g Fp.: 122 - 123 °C, Basenzahl: 170 mg KOH/g) aus und wird abfiltriert. Das Filtrat wird zur Trockene eingeengt.

| | |
|---|---|
| Ausbeute: | 67,4 g (84 % Theorie) |
| Schmelzbereich: | 95 - 171 °C |
| Stickstoffgehalt: | 3,25 % |
| Carboxyl-Zahl: | 132,0 mg KOH/g |
| Reinheit: | 92,7 % Decylglucaminoessigsaure-Na (¹³C-NMR) |
| | 2,6 % Decylglucamin |
| | 4,2 % Na-Glykolat |
| | 0,51 % NaCl |

### 3. Dodecylglucaminoessigäsure-Na-Salz

Analog Beispiel 2 wird Chloressigsäure mit Dodecylglucamin umgesetzt.

| | |
|---|---|
| Ausbeute: | 71,5 g (83,3 % Theorie) |
| Schmelzpunkt: | 172 - 174 °C |
| Stickstoffgehalt: | 3,07 % |
| Carboxyl-Zahl: | 115,3 mg KOH/g |
| Reinheit: | 96,0 % Dodecylglucaminoessigsäure-Na (¹³C-NMR) |
| | 2,4 % Na-Glykolat |
| | 1,6 % NaCl |

### 4. Tetradecylglucaminoessigsäure-Na-Salz

Analog Beispiel 2 wird Chloressigsäure mit Tetradecylglucamin umgesetzt.

| | |
|---|---|
| Ausbeute: | 94,9 g (97,8 % Theorie) |
| Schmelzbereich: | 160 - 162 °C |
| Stickstoffgehalt: | 2,88 % |
| Carboxyl-Zahl: | 111,6 mg KOH/g |
| Reinheit: | 97,8 % Tetradecylglucaminoessigsäure-Na (¹³C-NMR) |
| | 1,8 % Na-Glykolat |
| | 0,4 % NaCl |

### 5. Octadecylglucaminoessigsäure-Na-Salz

Analog Beispiel 2 wird Chloressigsäure mit Octadecylglucamin umgesetzt.

| | |
|---|---|
| Ausbeute: | 91,8 g (89,5 % Theorie) |
| Schmelzbereich: | 75 - 132 °C, 210 °C Zers. |
| Stickstoffgehalt: | 3,72 % |
| Reinheit: | 83,6 % Octadecylglucaminoessigsäure-Na (¹³C-NMR) |
| | 12,8 % Octadecylglucamin |
| | 3,3 % Na-Glykolat |
| | 0,30 % NaCl |

### Beispiele zu Eigenschaften hinsichtlich der Verwendung als Tensid

### 6. Grenz-/Oberflächenspannung und krit. Micellbildungskonzentration

Es wurden die nach den Herstellungsbeispielen synthetisierten Alkylglucaminoacetate mit n = 8, 10, 12, 14, 16 und 18 als 1%ige wäßrige Lösungen nach Einstellung auf pH 5,6 vermessen.

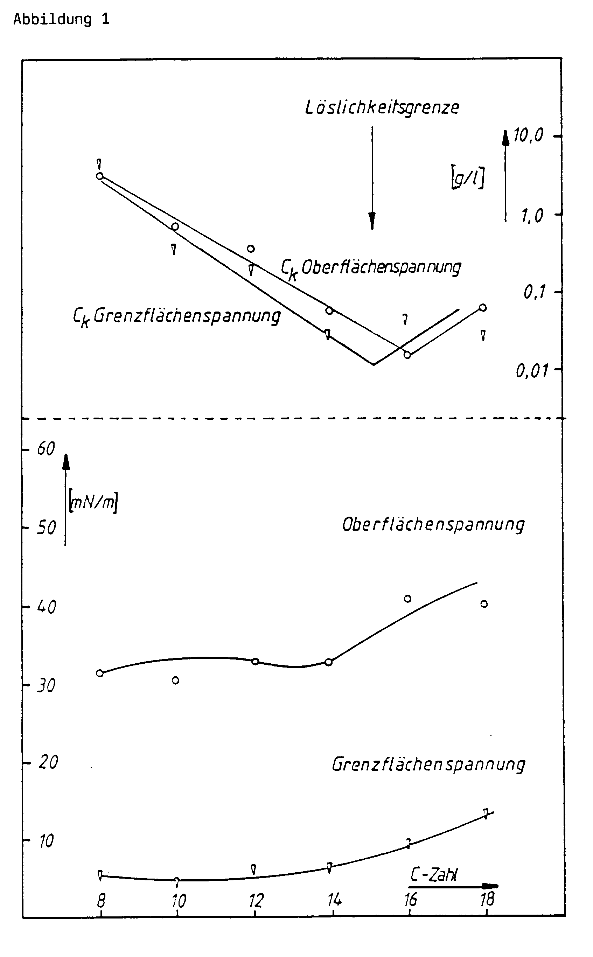

Abbildung 1 zeigt die Ergebnisse der Messung der Grenzflächenspannung und der Oberflächenspannung (unterer Teil der Abbildung) sowie der kritischen Micellbildungskonzentration mittels Lauda-Tensiometer (oberer Teil der Abbildung), gemessen gegen Luft bzw. Paraffin nach der Ringmethode du Noüy.

In ihren tensidischen Eigenschaften zeigen die Glucaminoacetate in bezug auf die Erniedrigung der Ober- und der Grenzflächenspannung klassisches Verhalten.

Zum Vergleich wurden die Ober- und Grenzflächenspannung sowie die kritische Micellbildungskonzentration des Carboxymethyldimethylkokosfettammoniumbetains (R-N⁺(CH₃)₂-CH₂COO⁻ mit R = C₁₂-C₁₄), einem marktgängigen Betain, ermittelt (siehe Tabelle 1).

**Tabelle 1**

| Grenz-/Oberflächenspannung und kritische Micellbildungskonzentration (cₖ-Wert) | | | | |
|---|---|---|---|---|
| | Oberflächenspannung (20 °C; in VE-Wasser) | | Grenzflächenspannung (20 °C, gegen Paraffin) | |
| | cₖ (g/l) | OF-SP (mN/m) | cₖ (g/l) | GF-SP (mN/m) |
| Dodecylglucaminoessigsäure | 0,37 | 33,0 | 0,22 | 5,8 |
| Tetradecylglucaminoessigsäure | 0,058 | 32,8 | 0,03 | 5,8 |
| Betain | 0,04 | 33,2 | 0,61 | 3,2 |
| VE = Vollentsalztes Wasser | | | | |

### 7. Schaumvermögen

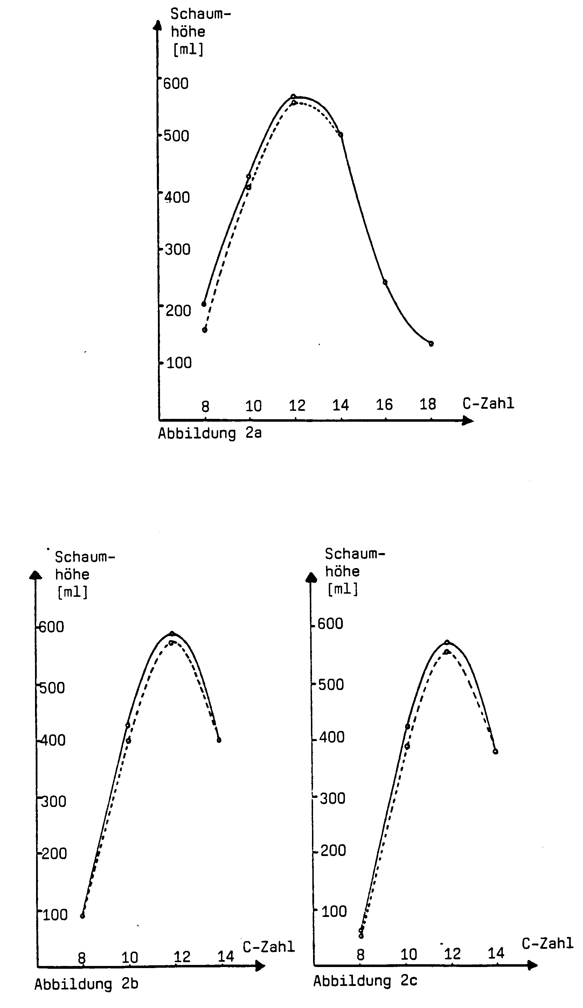

Die in dem Beispiel 6 verwendeten Alkylglucaminoacetate wurden als 0,1%ige wäßrige Lösung bei den pH-Einstellungen 9,9 ± 0,3; 7,00 und 5,60 nach DIN 53 902 vermessen. Bei pH 7.00 und 5,60 zeigen die C₁₆-und C₁₈-Glucaminoessigsäuren keine ausreichende Löslichkeit. Die Ergebnisse sind in den Abbildungen 2a (für pH 9,9 ± 0,3), 2b (für pH 7,00) und 2c (für pH 5,60) dargestellt. Die durchgezogene Kurve bedeutet jeweils die Werte nach 30 Sekunden und die gestrichelte Kurve die Werte nach 300 Sekunden.

Das maximale Schaumvermögen wurde mit dem Dodecylglucaminoacetat erreicht. Die erzielten Werte (Ablesung nach 30 s/300 s) von 580 ml/560 ml sind vergleichbar mit denen des kommerziellen Aniontensids (MARLON^{R} A: Alkylbenzolsulfonat) von 590 ml/570 ml und liegen deutlich höher als die des Ethersulfat (MARLINAT^{R} 242: C₁₂₋₁₄ mit 2,2-EO) mit 540 ml/540 ml und des Carboxymethyldimethylkokosfettammoniumbetains mit 510 ml/500 ml.

Eine pH-Wert-Abhängigkeit läßt sich trotz des ampholytischen Charakters der Alkylglucaminoverbindungen nicht beobachten.

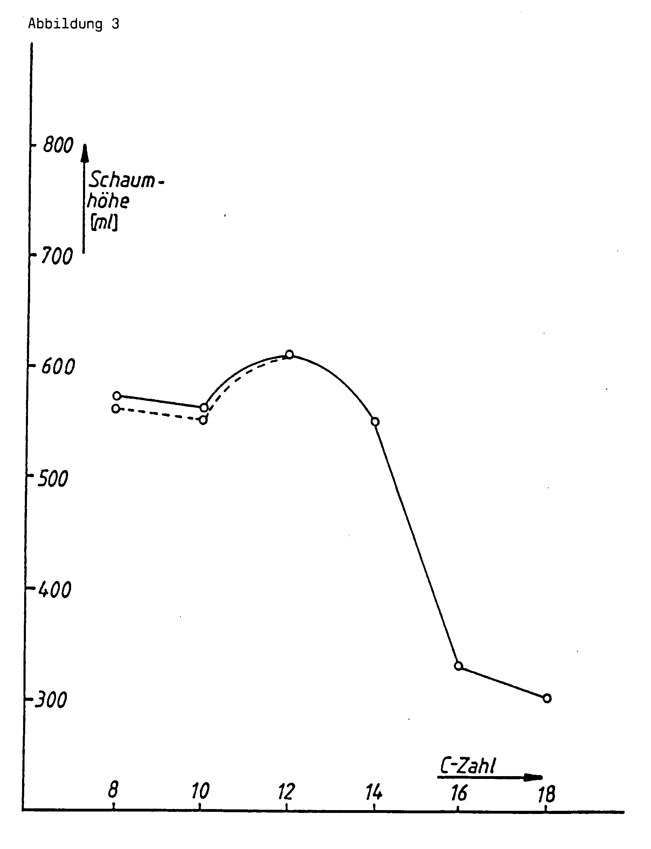

Durch Abmischen der Alkylglucaminoverbindungen mit dem Tensid MARLINAT^{R} 242 wird das Schaumvermögen der kurzkettigen Alkylglucaminoessigsäuren deutlich gesteigert. Das Maximum wird ebenfalls mit dem Dodecylglukaminoacetat erreicht (siehe Abbildung 3: gemessen bei pH 5,60, ― Werte nach 30 Sekunden, ----- Werte nach 300 Sekunden).

### 8. Benetzungsvermögen am Haar

Der Einsatz eines Tensides in Shampoos setzt eine gute Benetzung des Haares voraus. Zur Beurteilung der Benetzung wurde der Randwinkel zwischen einem zum Teil eingetauchten Haar und der Oberfläche der Tensidlösung herangezogen [Colloids and Surfaces 6 (1983) 49 - 61].

Das Dodecylglucaminoessigsäure-Na-Salz zeigt als 0,1%ige Tensidlösung bei pH 6, bezogen auf Wasser, eine Randwinkelerniedrigung von 53,8 %.

### 9. Hautverträglichkeit

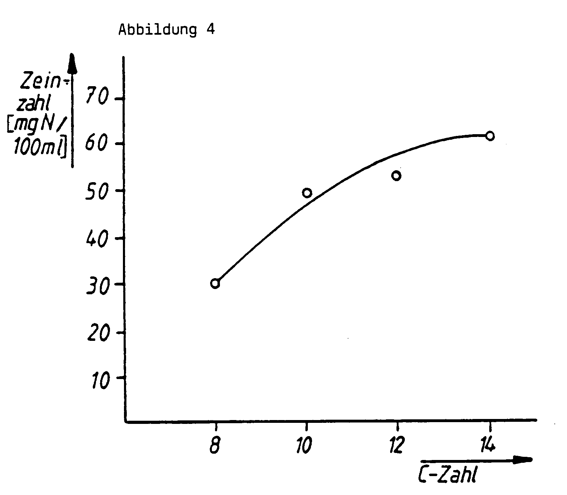

Die Hautverträglichkeit wurde durch den Zein-Test nach Götte (CID Brüssel 1984, 83 -90) ermittelt. Abbildung 4 zeigt die Ergebnisse für die Alkylglucaminoessigsäure-Na-Salze der Beispiele 1 bis 4 in 1%iger wäßriger Lösung bei pH 5,6.

Die ermittelten Zein-Zahlen lassen auf eine sehr gute Hautverträglichkeit schließen. Sie liegen deutlich unter dem Wert des kommerziellen Aniontensids MARLON^{R} A von 510 mg N/100 ml und sind vergleichbar mit denen der Betaine, mit denen Zein-Zahlen um 50 mg N/100 ml erreicht werden.

## Patentansprüche

1. N-Alkylglykaminoverbindungen der allgemeinen Formel IV 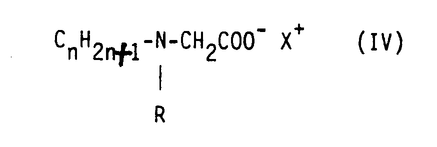 in der
n 4 - 24, und 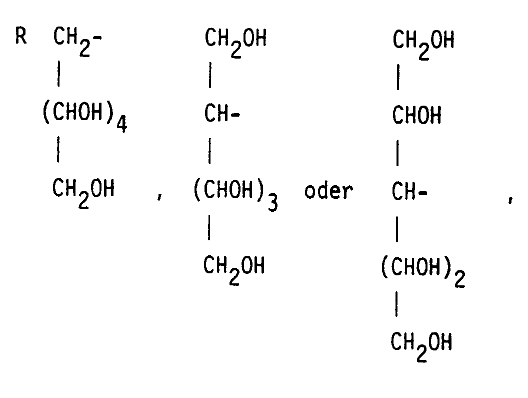 und
X Na, K oder H bedeuten.

2. N-Alkylglykaminoverbindungen der allgemeinen Formel IV nach Anspruch 1,
dadurch gekennzeichnet,
daß n 8 - 14 bedeuten.

3. N-Alkylglykaminoverbindungen der allgemeinen Formel IV nach Anspruch 1,
dadurch gekennzeichnet,
daß R 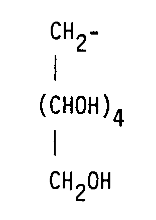 bedeutet.

4. N-Alkylglykaminoverbindungen der allgemeinen Formel IV nach Anspruch 1,
dadurch gekennzeichnet,
daß X Na bedeutet.

5. Verfahren zur Herstellung von N-Alkylglykaminoverbindungen nach den Ansprüchen 1 bis 4,
dadurch gekennzeichnet,
daß die entsprechenden N-Alkylglykamine bei pH 8 bis 9 und T < 80 °C in an sich bekannter Weise mittels Chloressigsäure N-carboxymethyliert werden durch Zugabe von Alkalihydroxid.

6. Verfahren zur Herstellung von N-Alkylglykaminoverbindungen nach Anspruch 5,
dadurch gekennzeichnet,
daß als Alkalihydroxid Natriumhydroxid verwendet wird.

7. Verwendung von N-Alkylglykaminoverbindungen nach Anspruch 1 als Tensid.

8. Verwendung von N-Alkylglykaminoverbindungen nach Anspruch 1 als Amphotensid.

9. Verwendung von N-Alkylglykaminoverbindungen nach Anspruch 1 in Körperpflegemitteln.

## Claims

1. An N-alkylglycamino compound of the general formula IV 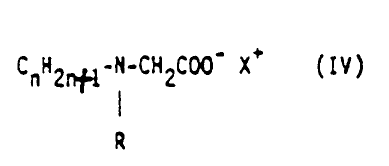 in which
n denotes 4 - 24 and
R denotes 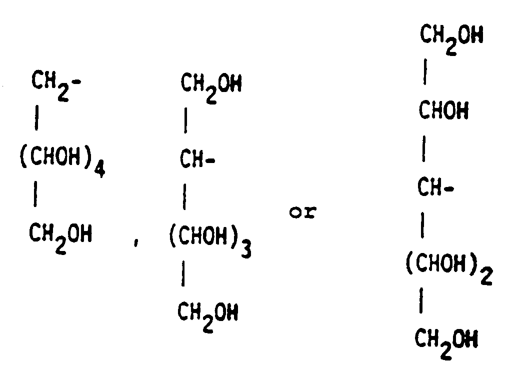 and X denotes Na, K or H.

2. An N-alkylglycamino compound of the general formula IV according to Claim 1, characterized in that n denotes 8 - 14.

3. An N-alkylgycamino compound of the general formula IV according to Claim 1, characterized in that R denotes

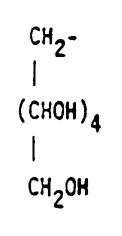

4. An N-alkylglycamino compound of the general formula IV according to Claim 1, characterized in that X denotes Na.

5. A process for the preparation of an N-alkylglycamino compound according to any of Claims 1 to 4, characterized in that the corresponding N-alkylglycamine is N-carboxymethylated at pH 8 to 9 and T < 80°C in a manner known per se by means of chloroacetic acid, by addition of alkali metal hydroxide.

6. A process for the preparation of an N-alkylglycamino compound according to Claim 5, characterized in that sodium hydroxide is used as the alkali metal hydroxide.

7. The use of an N-alkylglycamino compound according to Claim 1 as a surfactant.

8. The use of an N-alkylglycamino compound as claimed in claim 1 as an amphosurfactant.

9. The use of an N-alkylglycamino compound according to Claim 1 in body care agents.

## Revendications

1. Les N-alkyl-glycamino-composés de la formule générale (IV) 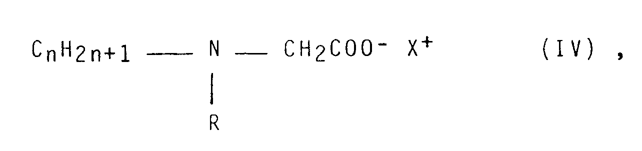 dans laquelle n a une valeur de 4 à 24, et R représente 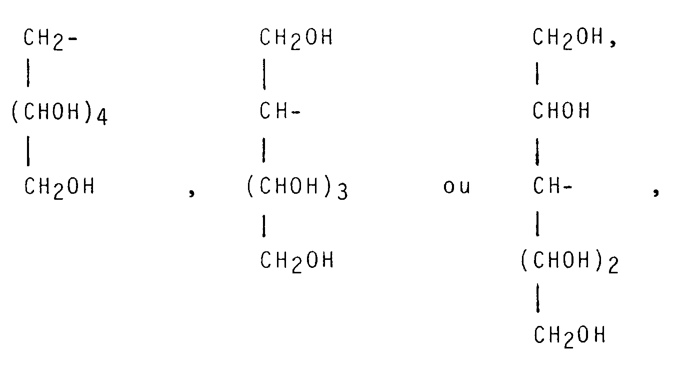 et X est le sodium, le potassium ou l'hydrogène.

2. Les N-alkyl-glycamino-composés de la formule générale (IV) selon la revendication 1,
caractérisés par le fait que n a une valeur de 8 à 14.

3. Les N-alkyl-glycamino-composés de la formule générale (IV) selon la revendication 1,
caractérisés par le fait que R répond à la formule

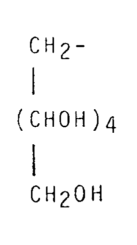

4. Les N-alkyl-glycamino-composés de la formule générale (IV) selon la revendication 1,
caractérisés par le fait que X représente le sodium.

5. Procédé de préparation de N-alkyl-glycamino-composés selon les revendications 1 à 4,
caractérisé par le fait qu'à un pH de 8 à 9 et à une température inférieure à 80°C, on soumet d'une manière connue en soi les N-alkyl-glycamines correspondantes à une N-carboxyméthylation à l'aide d'acide chloracétique, en ajoutant un hydroxyde alcalin.

6. Procédé de préparation de N-alkyl-glycamino-composés selon la revendication 5,
caractérisé par le fait que l'on utilise l'hydroxyde de sodium comme hydroxyde alcalin.

7. L'utilisation, en tant que surfactifs, de N-alkyl-glycamino-composés selon la revendication 1.

8. L'utilisation, en tant qu'ampho-surfactifs, de N-alkyl-glycamino-composés selon la revendication 1.

9. L'utilisation, en tant qu'agents pour soins corporels, de N-alkyl-glycamino-composés selon la revendication 1.
